# FASCICULE DE BREVET EUROPEEN

(11) **EP 1 464 318 B1**
(45) Date de publication et mention de la délivrance du brevet: **11.10.2006**
(21) Numéro de dépôt: 04290866.5
(22) Date de dépôt: 01.04.2004
(51) Int. Cl.: A61K 8/41, A61K 8/49, A61Q 5/04, A61Q 5/10

(54) **Procédé de coloration avec effet eclaircissant de fibres keratiniques humaines ayant subi une déformation permanente, au moyen d'une composition comprenant un colorant fluorescent**
Verfahren zum Färben mit aufhellender Wirkung von menschlichen Keratinfasern mit vorausgegangener Dauerwellbehandlung, mittels einer einen fluoreszierenden Farbstoff enthaltenden Zusammensetzung
Process for dyeing humain keratinic fibres with a brightening effect, wherein the fibres had undergone a permanent wave treatment and wherein a composition comprising a fluorescent dye is applied

(30) Priorité: 01.04.2003 FR 0304031
(43) Date de publication de la demande: 06.10.2004
(73) Titulaire: L'ORÉAL, 75008 Paris (FR)
(72) Inventeur: Plos, Grégory, 75011 Paris (FR); Samain, Henri, 91570 Bievres (FR)
(74) Mandataire: Wattremez, Catherine

(56) Documents cités:
- GB-A- 759 385
- US-A- 4 781 724

## Description

L'invention concerne un procédé de coloration de fibres kératiniques ayant subi préalablement un procédé de déformation permanente, en mettant en oeuvre une composition comprenant, dans un milieu cosmétiquement acceptable, au moins un colorant fluorescent soluble dans ledit milieu

Dans le domaine capillaire, il est fréquent de devoir mettre en oeuvre des étapes de mise en forme permanente et de coloration à peu d'intervalle de temps, voire immédiatement l'une après l'autre, l'opération de mise en forme étant réalisée en première étape. Cependant de tels procédés ne sont pas sans dommage pour les fibres kératiniques.

En effet, l'un des procédés classiques de déformation du cheveu consiste à procéder en deux étapes, la première consistant à réduire les ponts di-sulfures présents dans la fibre kératinique, en utilisant un agent réducteur. Une fois ces ponts di-sulfures réduits, le cheveu est alors mis en forme de la manière souhaitée. Celle-ci peut consister à friser le cheveu ou bien à le lisser, le résultat dépendant du moyen employé pour la mise sous tension. Cette opération de mise sous tension peut être effectuée avant, pendant ou après l'application de la composition réductrice. Une fois cette première étape réalisée, une étape d'oxydation est nécessaire pour recréer les ponts di-sulfures et stabiliser la forme obtenue. Cette opération est effectuée habituellement en milieu oxydant.

Il existe un autre procédé de déformation permanente du cheveu, applicable essentiellement dans le cadre d'un défrisage, et qui consiste habituellement à appliquer sur la chevelure, en la lissant, une solution d'hydroxyde de métal alcalin concentrée. Lors de cette opération, la fibre kératinique est relativement endommagée car solubilisée partiellement dans la solution alcaline. Une fois ce traitement effectué, les cheveux sont rincés.

Il est donc clair qu'après ce type de traitements, la fibre kératinique est relativement abîmée, fragilisée, et la mise en oeuvre d'une étape de coloration ultérieure représente un risque supplémentaire de dégradation, risque d'autant plus marqué que l'on souhaite obtenir un éclaircissement des fibres.

En effet, les procédés de coloration sont mis en oeuvre en présence d'un agent oxydant, en milieu alcalin. De plus, ces conditions sont d'autant plus dures que le degré souhaité d'éclaircissement de la chevelure est important.

La présente invention a donc pour but de proposer un procédé de coloration avec un effet d'éclaircissement de la chevelure, mis en ceuvre postérieurement à un procédé de déformation permanente des cheveux, qui ne contribue pas de manière importante à une dégradation nouvelle des fibres traitées.

Il a donc été trouvé de façon inattendue et surprenante que l'utilisation de colorants fluorescents, en particulier ceux dans la gamme des orangés, mis en oeuvre après un procédé de déformation permanente des fibres kératiniques, permettait d'obtenir des colorations esthétiques, homogènes, avec de bonnes propriétés de ténacité vis-à-vis des agents extérieurs, et en particulier des shampooings.

La présente invention a donc pour objet un procédé pour colorer avec un effet éclaircissant les fibres kératiniques humaines ayant subi antérieurement un procédé de déformation permanente, caractérisé en ce que l'on met en oeuvre les étapes suivantes :
a) on applique sur lesdites fibres, une composition comprenant, dans un milieu cosmétiquement acceptable, au moins un colorant fluorescent soluble dans le milieu, pendant un temps suffisant pour développer la coloration et l'éclaircissement désirés, le colorant fluorescent étant une molécule qui colore par elle-même, et donc absorbe la lumière du spectre visible, mais qui transforme l'énergie absorbée en lumière fluorescente de plus grande largeur d'onde émise dans la partie visible du spectre,
b) on rince éventuellement les fibres,
c) éventuellement on lave au shampooing et on rince les fibres,
d) on sèche ou on laisse sécher les fibres.

Mais d'autres caractéristiques et avantages de la présente invention apparaîtront plus clairement à la lecture de la description et de l'exemple qui vont suivre.

A moins d'une indication différente, les bornes des gammes de valeurs qui sont données dans la description, sont incluses dans ces gammes.

Pour des raisons de clarté dans l'exposé, la composition mise en oeuvre dans l'étape a) va tout d'abord être décrite.

Comme cela a été indiqué auparavant, la composition comprend au moins un colorant fluorescent soluble dans le milieu de la composition.

Par colorant fluorescent, on entend au sens de la présente invention un colorant qui est une molécule qui colore par elle-même, et donc absorbe la lumière du spectre visible et éventuellement de l'ultraviolet (longueurs d'onde allant de 360 à 760 nanomètres) mais qui, contrairement à un colorant classique, transforme l'énergie absorbée en lumière fluorescente de plus grande longueur d'onde émise dans la partie visible du spectre.

Un colorant fluorescent selon l'invention est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores, qui ne colorent pas, car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Enfin, le colorant fluorescent mis en oeuvre dans la composition est soluble dans le milieu de la composition. Précisons que le colorant fluorescent est différent en cela d'un pigment fluorescent qui lui, n'est pas soluble dans le milieu de la composition.

Plus particulièrement, le colorant fluorescent utilisé dans le cadre de la présente invention, éventuellement neutralisé, est soluble dans le milieu de la composition à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

Les colorants fluorescents utilisés de préférence selon la présente invention sont des colorants dans la gamme des orangés.

De préférence, les colorants fluorescents de l'invention conduisent à un maximum de réflectance se situant dans la gamme de longueur d'onde allant de 500 à 650 nanomètres, et de préférence dans la gamme de longueur d'onde allant de 550 à 620 nanomètres.

Certains des colorants fluorescents selon la présente invention sont des composés connus en eux-mêmes.

A titre d'exemples de colorants fluorescents susceptibles d'être mis en oeuvre, on peut citer les colorants fluorescents appartenant aux familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges, et de préférence aux familles suivantes : les naphtalimides ; les coumarines cationiques
ou non ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges.

On peut notamment citer parmi eux :
- les composés de structure suivante : 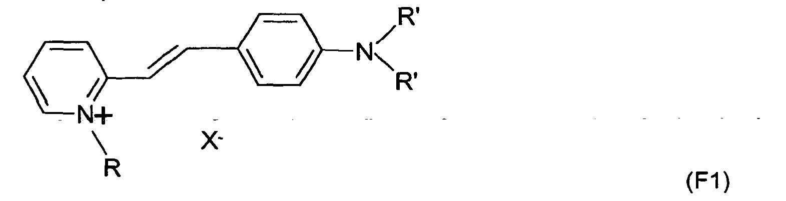 formule dans laquelle R représente un radical méthyle ou éthyle; R' représente un radical méthyle, X⁻ un anion du type iodure, sulfate, méthosulfate.
   A titre d'exemple de composé de ce type on peut citer le Photosensitiving Dye NK-557 commercialisé par la société UBICHEM, pour lequel R représente un radical éthyle, R' un radical méthyle et X- un iodure.
- le Jaune Brilliant B6GL commercialisé par la société SANDOZ et de structure suivante : 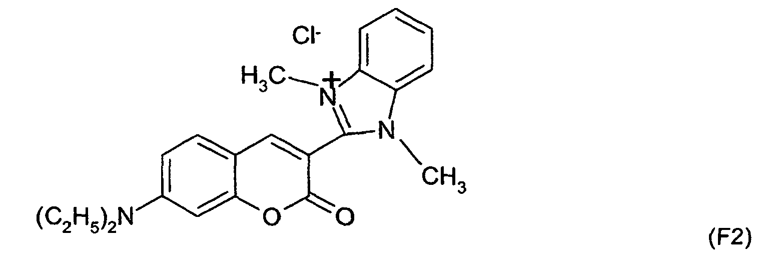
- le Basic Yellow 2, ou Auramine O commercialisé par les sociétés PROLABO, ALDRICH
ou CARLO ERBA et de structure suivante : 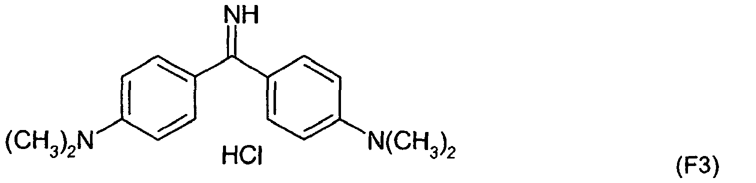 monochlorhydrate de 4,4'-(imidocarbonyl)bis(N,N-diméthylaniline) - CAS number 2465-27-2.

On peut aussi citer les composés de formule suivante : 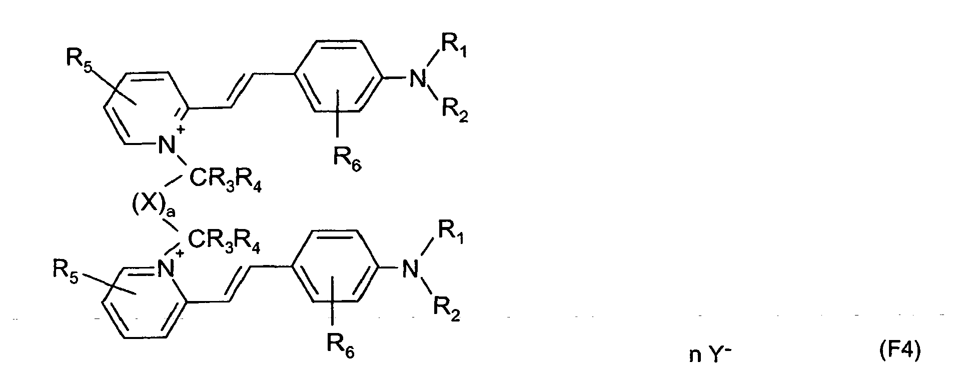 dans laquelle :
R₁, R₂, identiques ou différents, représentent :
   - un atome d'hydrogène ;
   - un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
   - un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
   - R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
   - R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
   - un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
   - un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
   - un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
   - un radical dicarbonyle ;
   - le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

Rappelons que les termes hétéroatomes, représentent un atome d'oxygène ou d'azote.
Parmi les groupements porteurs de tels atomes, on peut citer entre autres les groupements hydroxyle, alcoxy, carbonyle, amino, ammonium, amido (-N-CO-), carboxyle (-O-CO- ou -CO-O-).
En ce qui concerne les groupements alcényles, ces derniers comprennent une ou plusieurs liaisons carbone-carbone insaturée (-C=C-), et de préférence une seule double liaison carbone-carbone.

Dans cette formule générale, les radicaux R₁ et R₂, identiques ou non, représentent plus particulièrement :
- un atome d'hydrogène ;
- un radical alkyle comprenant 1 à 10 atomes de carbone, notamment 1 à 6 atomes de carbone, de préférence 1 à 4 atomes de carbone, éventuellement interrompu par un atome d'oxygène ou éventuellement substitué par au moins un radical hydroxyle, amino, ammonium, d'un atome de chlore ou de fluor;
- un radical benzyle, phényle, éventuellement substitué par un radical alkyle ou alcoxy comprenant 1 à 4 atomes de carbone, de préférence 1 ou 2 atomes de carbone ;
- avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire ou ramifié comprenant 1 à 4 atomes de carbone éventuellement interrompu et/ou substitué par un atome d'azote et/ou d'oxygène et/ou groupement portant un atome d'azote et/ou d'oxygène.

En ce qui concerne les radicaux amino ou ammonium précités, les radicaux portés par l'atome d'azote peuvent ou non être identiques et représenter plus particulièrement un atome d'hydrogène, un radical alkyle en C₁-C₁₀, de préférence en C₁-C₄, un radical arylalkyle dans lequel, plus spécialement, le radical aryle comprend 6 atomes de carbone et le radical alkyle 1 à 10 atomes de carbone, de préférence 1 à 4 atomes de carbone.

Selon un mode de réalisation avantageux de l'invention, les radicaux R₁ et R₂, identiques ou non, représentent un atome d'hydrogène ; un radical alkyle linéaire ou ramifié en C₁-C₆ ; un radical alkyle en C₂-C₆ substitué par un radical hydroxyle ; un radical alkyle en C₂-C₆ portant un groupement amino ou ammonium ; un radical chloroalkyle en C₂-C₆ ; un radical alkyle C₂-C₆ interrompu par un atome d'oxygène ou groupement en portant un (par exemple ester) ; un radical aromatique comme les phényle, benzyle, 4-méthylphényle ; un radical hétérocyclique tel que les radicaux pyrrolo, pyrrolidino, imidazolo, imidazolino, imidazolium, pipérazino, morpholo, morphollino, pyrazolo, triazolo, éventuellement substitué par au moins un radical alkyle en C₁-C₆ ou aromatique.

De préférence, les radicaux R₁ et R₂, identiques ou différents, représentent un atome d'hydrogène, un radical alkyle linéaire ou ramifié en C₁-C₆ tels que les radicaux méthyle, éthyle, n-butyle, n-propyle ; le 2-hydroxyéthyle ; un radical alkyltriméthylammonium ou alkyltriéthylammonium, le radical alkyle étant linéaire en C₂-C₆ ; un radical (di)alkylméthylamino ou (di)alkyléthylamino, le radical alkyle étant linéaire en C₂-C₆ ; -CH₂CH₂Cl ; -(CH₂)ₙ-OCH₃ ou -(CH₂)ₙ-OCH₂CH₃ avec n nombre entier variant de 2 à 6 ; -CH₂CH₂-OCOCH₃ ; -CH₂CH₂COOCH₃.
De préférence les radicaux R₁ et R₂, identiques ou non, et de préférence identiques, représentent un radical méthyle, un radical éthyle.

Les radicaux R₁ et R₂, identiques ou différents, peuvent aussi représenter un radical hétérocyclique du type pyrrolidino, 3-amino pyrrolidino, 3-(diméthyl)amino pyrrolidino, 3-(triméthyl)amino pyrrolidino, 2,5-diméthylpyrrolo, le 1H-imidazole, 4-méthyl pipérazino, 4-benzyl pipérazino, morpholo, 3,5-(ter-butyl)-1H-pyrazolo, 1H-pyrazolo, 1H-1,2,4-triazolo.

Les radicaux R₁ et R₂, identiques ou différents, peuvent aussi représenter être reliés de manière à former un hétérocycle de formules (I) et (II) suivantes : 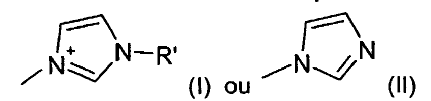 dans lesquelles R' représente un atome d'hydrogène, un radical alkyle en C₁-C₃, -CH₂CH₂OH, -CH₂CH₂OCH₃.

Conformément à un mode de réalisation plus particulier de l'invention, R₅, identiques ou non, représentent un atome d'hydrogène, un atome de fluor ou de chlore, un radical alkyle linéaire ou ramifié comprenant 1 à 4 atomes de carbone éventuellement interrompu par un atome d'oxygène ou d'azote.
Il est précisé que le substituant R₅, s'il est différent de l'hydrogène, se trouve avantageusement en position(s) 3 et/ou 5 par rapport au carbone du cycle portant l'azote substitué par les radicaux R₁ et R₂, et de préférence en position 3 par rapport à ce carbone.
Avantageusement, les radicaux R₅, identiques ou non, représentent un atome d'hydrogène ; un radical alkyle linéaire ou ramifié en C₁-C₄ ; -O-R₅₁ avec R₅₁ représentant un radical alkyle linéaire en C₁-C₄ ; -R₅₂-O-CH₃ avec R₅₂ représentant un radical alkyle linéaire en C₂-C₃ ; -R₅₃ - N (R₅₄)₂ dans laquelle R₅₃ représente un radical alkyle linéaire en C₂-C₃, R₅₄, identiques ou différents, représentent un atome d'hydrogène ou un radical méthyle.
De préférence R₅, identiques ou non, représentent l'hydrogène, un méthyle, un méthoxy, et de préférence, R₅ représente un atome d'hydrogène.

Selon un mode de réalisation particulier, les radicaux R₆, identiques ou différents, représentent un atome d'hydrogène ; un radical alkyle linéaire ou ramifié en C₁-C₄ ; -X avec X représentant un atome de chlore, de brome ou de fluor ; -R₆₁-O-R₆₂ avec R₆₁ représentant un radical alkyle linéaire en C₂-C₃ et R₆₂ représente le radical méthyle ; -R₆₃-N(R₆₄)₂ avec R₆₃ représentant un radical alkyle linéaire en C₂-C₃, R₆₄, identiques ou différents, représentent un atome d'hydrogène ou un radical méthyle, -N(R₆₅)₂ dans laquelle R₆₅, identiques ou différents, représentent un atome d'hydrogène, un radical alkyle linéaire en C₂-C₃ ; -NHCO R₆₆ avec R₆₆ représentant un radical alkyle en C₁-C₂, un radical chloroalkyle en C₁-C₂, un radical -R₆₇-NH₂ ou -R₆₇-NH(CH₃) ou -R₆₇-N(CH₃)₂ ou -R₆₇-N⁺(CH₃)₃ ou -R₆₇-N⁺(CH₂CH₃)₃ avec R₆₇ représentant un radical alkyle en C₁-C₂.

Il est précisé que le substituant R₆, s'il est différent de l'hydrogène, se trouve de préférence en position 2 et/ou 4 par rapport à l'atome d'azote du cycle pyridinium, et de préférence en position 4 par rapport à cet atome d'azote.

Plus particulièrement ces radicaux R₆, identiques ou non, représentent un atome d'hydrogène ou un radical méthyle ou éthyle, et de préférence, R₆ représente un atome d'hydrogène.

En ce qui concerne les radicaux R₃, R₄, ces derniers, identiques ou non, représentent avantageusement un atome d'hydrogène, un radical alkyle comprenant 1 à 4 atomes de carbone, plus spécialement un radical méthyle. De manière préférée, R₃ et R₄ représentent chacun un atome d'hydrogène.

Comme indiqué plus haut, X représente :
- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome, par au moins un groupement porteur d'au moins un hétéroatome et/ou par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, par au moins un radical aminoalkyle, linéaire ou ramifié, comprenant 1 à 4 atomes de carbone, éventuellement substitué par au moins un hétéroatome ; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement comprenant au moins un hétéroatome ;
- un radical dicarbonyle.
En outre, il est indiqué que le groupement X peut porter une ou plusieurs charges cationiques.

Ainsi, X peut représenter un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome , et/ou par un atome de fluor, de chlore.
Parmi les groupements de ce type, on peut citer tout particulièrement les groupements hydroxyle, alcoxy (avec notamment un radical R de type alkyle en C₁-C₄), amino, ammonium, amido, carbonyle, carboxyle (-COO-, -O-CO-) avec notamment un radical de type alkyloxy.
Notons que l'atome d'azote, s'il est présent, peut se trouver sous une forme quaternisée ou non. Dans ce cas, le ou les deux autres radicaux portés par l'atome d'azote quaternisé ou non, sont identiques ou non et peuvent être un atome d'hydrogène, un radical alkyle en C₁-C₄, de préférence le méthyle.

Selon une autre variante, le groupement X représente un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome (de préférence un radical hydroxyle), ou par un atome d'halogène. A noter que le groupement amino est de préférence lié à l'hétérocycle.

Conformément à une autre possibilité, le groupement X représente un radical aromatique (comprenant de préférence 6 atomes de carbone) ou diaromatique condensé ou non (comprenant notamment de 10 à 12 atomes de carbone), séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone, de préférence 1 à 4 atomes de carbone, éventuellement interrompu par au moins un atome d'oxygène et/ou d'azote, et/ou groupement comprenant au moins un hétéroatome (comme un radical carbonyle, carboxyle, amido, amino, ammonium).
Il est à noter que le radical aromatique, de préférence un radical phényle, est relié aux groupements CR₃R₄ par l'intermédiaire de liaisons en positions 1,2 ; 1,3 ou 1;4, de préférence en positions 1,3 et 1,4. Si le radical phényle relié par l'intermédiaire de liaisons en positions 1,4, porte un ou deux substituants, ce ou ces derniers sont situés de préférence en position 1,4 par rapport à l'un des groupements CR₃R₄. Si le radical phényle relié par l'intermédiaire de liaisons en positions 1,3, porte un ou deux substituants, ce ou ces derniers sont situés de préférence en position 1 et/ou 3 par rapport à l'un des groupements CR₃R₄,

Au cas où le radical est diaromatique, il est de préférence non condensé et comprend deux radicaux phényles séparés ou non par une liaison simple (soit un carbone de chacun des deux cycles) ou par un radical alkyle, de préférence de type CH₂ ou C(CH₃)₂. De manière préférée, les radicaux aromatiques ne portent pas de substituant.
Il est à noter que ledit radical diaromatique est relié aux groupements CR₃R₄ par l'intermédiaire de liaisons en positions 4,4'.

A titre d'exemples de groupements X convenables, on peut citer notamment les radicaux alkyle linéaires ou ramifiés comprenant 1 à 13 atomes de carbone tels que méthylène, éthylène, propylène, isopropylène, n-butylène, pentylène, hexylène ; le 2-hydroxypropylène, le 2-hydroxy n-butylène ; les radicaux alkylènes en C₁-C₁₃, substitués ou interrompus par un ou plusieurs atomes d'azote et/ou d'oxygène, et/ou groupements portant au moins un hétéroatome (hyroxyle, amino, ammonium, carbonyle, carboxyle, par exemple) tels que -CH₂CH₂OCH₂CH₂-, le 1,6-didéoxy-d-mannitol, -CH₂N⁺(CH₃)₂CH₂-, -CH₂CH₂N⁺(CH₃)₂-(CH₂)₆N⁺(CH₃)₂-CH₂CH₂-, CO-CO-, le 3,3-diméthylpentylène, le 2-acétoxyéthylène, le butylène1,2,3,4 tétraol ; -CH=CH- ; les radicaux aromatiques ou diaromatiques substitués par un ou plusieurs radicaux alkyle, par un ou plusieurs groupements portant au moins un hétéroatome et/ou par un ou plusieurs atomes d'halogène, tels que le 1,4-phénylène, le 1,3-phénylène, le 1,2-phénylène, le 2,6-fluorobenzène, le 4,4'-biphénylène, le 1,3-(5-méthyl benzène), le 1,2-bis(2-méthoxy)benzène, le bis(4-phényl)méthane, le 3,4 benzoate de méthyle, le 1,4-bis(amido méthyl)phényle; les radicaux de type hétérocycliques comme la pyridine, ou dérivé tel que le 2,6-bispyridine, l'imidazole, l'imidazolium, la triazine.

X représente, selon un mode de réalisation plus particulier de l'invention, un radical alkyle linéaire ou ramifié en C₁-C₁₃ ; -CH₂CH(OH)CH₂- ; -CH₂CH(Cl)CH₂- ; -CH₂CH₂-OCOCH₂- ; -CH₂CH₂COOCH₂- ; -Ra-O- Rb- avec Ra représentant un radical alkyle linéaire en C₂-C₆ et Rb représente un radical alkyle linéaire en C₁-C₂ ; - Rc-N(Rd)-Re-avec Rc représentant un radical alkyle en C₂-C₉, Rd représentant un atome d'hydrogène, un radical alkyle en C₁-C₂ et Re représentant un radical alkyle en C₁-C₆ ; -Rf-N⁺(Rg)₂-Rh- avec Rf représentant un radical alkyle linéaire en C₂-C₉, Rg, de préférence identiques, représentent un radical alkyle en C₁-C₂, Rh représente un radical alkyle linéaire en C₁-C₆ ; -CO-CO-.

X peut de plus représenter un radical imidazole, éventuellement substitué par au moins un radical alkyle comprenant 1 à 14 atomes de carbone, plus particulièrement 1 à 10 atomes de carbone, de préférence de 1 à 4, et par exemple les radicaux divalents de formule suivante : 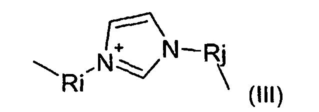
dans laquelle Ri et Rj, identiques ou non, représentent un radical alkyle linéaire en C₁-C₆;
X peut de même être choisi parmi les radicaux divalents dérivés de triazine suivants :

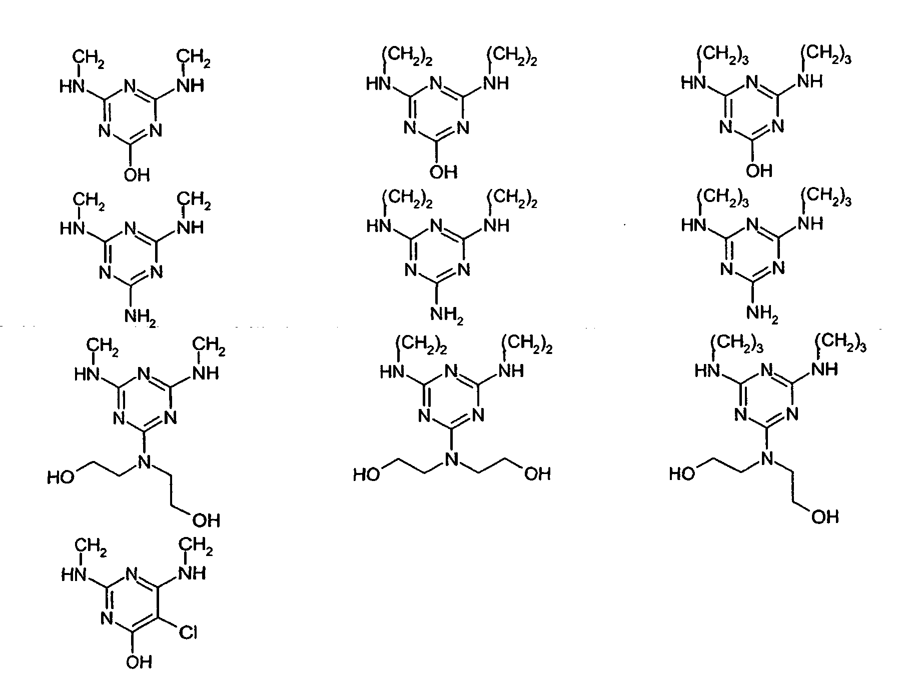

Selon une autre possibilité, X peut représenter les radicaux divalents aromatiques suivants :

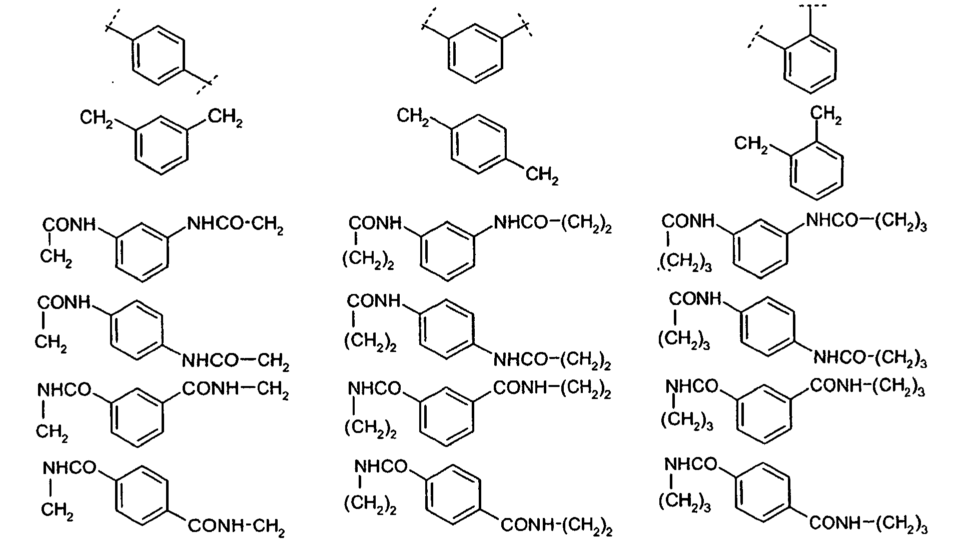

Dans la formule générale de ces composés fluorescents, Y- représente un anion organique ou minéral. S'il y a plusieurs anions Y-, ces derniers peuvent ou non être identiques.

Parmi les anions d'origine minérale, on peut citer sans intention de s'y limiter les anions provenant d'atomes d'halogène, tels que les chlorures de préférence, les iodures, les sulfates ou bisulfates, les nitrates, les phosphates, les hydrogénophosphates, les dihydrogénophosphates, les carbonate, les bicarbonates.
Parmi les anions d'origine organique, on peut citer les anions provenant des sels d'acides mono- ou polycarboxyliques, sulfoniques, sulfuriques, saturés ou non, aromatiques ou non, éventuellement substitués par au moins un radical hydroxyle, amino, ou atomes d'halogène. A titre d'exemples non limitatifs, conviennent les acétates, hydroxyactétates, aminoacétates, (tri)chloroacétates, benzoxyactétates, propionates et dérivés portant un atome de chlore, fumarates, oxalates, acrylates, malonates, succinates, lactates, tartrates, glycollates citrates, les benzoates et dérivés portant un radical méthyle ou amino, les alkylsulfates, les tosylates, les benzènesulfonates, toluènesulfonates, etc.
De préférence, le ou les anions Y, identiques ou non, sont choisis parmi le chlore, le sulfate, le méthosulfate, l'éthosulfate.

Enfin, le nombre n, entier, est au moins égal à 2 et au plus égal au nombre de charges cationiques présentes dans le composé fluorescent.

De préférence les composés fluorescents qui viennent d'être détaillés sont des composés symétriques.

Ces composés peuvent être synthétisés en mettant en faisant réagir dans une première étape de α-picoline avec un réactif comprenant deux groupes partant qui peuvent être choisis parmi les atomes d'halogène, de préférence le brome, éventuellement le chlore,
ou les groupements de type tolylsulfonyle ou méthylesulfonyle.
Cette première étape peut avoir lieu en présence d'un solvant, bien qu'il ne soit pas obligatoire, comme par exemple le diméthylformamide.
Le nombre de moles d'α-picoline est en général voisin de 2 pour une mole de réactif comprenant les groupes partant.
En outre, la réaction est habituellement mise en oeuvre au reflux du réactif et/ou du solvant s'il est présent.
Le produit issu de cette première étape est ensuite contacté avec un aldéhyde correspondant de formule suivante : 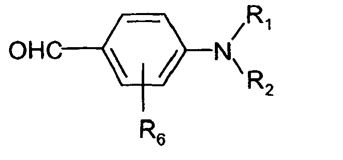 dans laquelle R₁, R₂ et R₆ ont les mêmes significations que précédemment indiquées.

Là encore, la réaction peut être effectuée en présence d'un solvant approprié, de préférence au reflux.
Il est à noter que les radicaux R₁ et R₂ de l'aldéhyde peuvent avoir la signification indiquée dans la formule générale détaillée auparavant.
Il est aussi possible de mettre en oeuvre un aldéhyde pour lequel lesdits radicaux représentent des atomes d'hydrogène et effectuer conformément à des méthodes classiques, la substitution de ces atomes d'hydrogène par des radicaux appropriés tels que décrits dans la formule générale une fois la deuxième étape terminée.

On pourra notamment se référer à des synthèses telles que décrites dans US 4256458.

Le ou les colorants fluorescents présents dans la composition selon l'invention représentent avantageusement de 0,01 à 20 % en poids, plus particulièrement de 0,05 à 10 % en poids, et de préférence de 0,1 à 5% en poids, du poids total de la composition.

Le milieu cosmétiquement acceptable est généralement constitué par de l'eau ou par un mélange d'eau et d'un ou plusieurs solvants organiques usuels.

Parmi les solvants convenables, on peut citer plus particulièrement, les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

Les solvants usuels décrits ci-dessus, s'ils sont présents, représentent habituellement de 1 à 40 % en poids, plus préférentiellement de 5 à 30 % en poids, par rapport au poids total de la composition.

Le pH de la composition est généralement compris entre 3 et 12 environ, et de préférence entre 5 et 11 environ.
Il peut être ajusté à la valeur désirée au moyen d'agents acidifiants ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 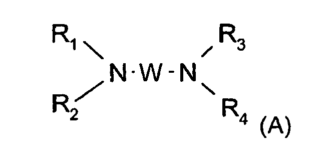 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ ou hydroxyalkyle en C₁-C₆.

Selon un mode de réalisation particulier de l'invention, la composition peut, comprendre, en plus du ou des colorants fluorescents, un ou plusieurs colorants directs additionnels non fluorescents de nature non ionique, cationique ou anionique, qui peuvent par exemple être choisis parmi les colorants benzéniques nitrés.
Conviennent notamment les colorants directs benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine, et
- le1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine, et
- le 1-amino-2-nitro-4-hydroxy-5-méthylbenzène.

La composition peut également comprendre, en addition ou en remplacement de ces colorants benzéniques nitrés, un ou plusieurs colorants directs additionnels choisis parmi les colorants benzéniques nitrés jaunes, jaune-vert, bleus ou violets, les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ainsi que les colorants dérivés du triarylméthane, ou leurs mélanges.

Ces colorants directs additionnels peuvent notamment être des colorants basiques parmi lesquels on peut citer plus particulièrement les colorants connus dans le COLOR INDEX, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le COLOR INDEX, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans WO 95/01772, WO 95/15144 et EP 714954 et dont le contenu fait partie intégrante de la présente invention.

Parmi les colorants directs additionnels benzéniques nitrés jaunes et jaune-vert, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène, et
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ dihydroxypropyl)amino 4-(N-éthyl, N-β-hydroxyéthyl)amino 2-nitrobenzène,
- les 2-nitroparaphénylènediamines de formule suivante : 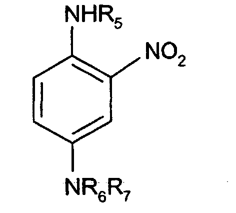
dans laquelle :
- R₆ représente un radical alkyle en C₁-C₄, un radical β-hydroxyéthyle ou β-hydroxypropyle ou γ-hydroxypropyle ;
- R₅ et R₇, identiques ou différents, représentent un radical β-hydroxyéthyle, -β-hydroxypropyle, γ-hydroxypropyle, ou β,γ dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans FR 2 692 572.

Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

La composition peut également comprendre divers adjuvants utilisés classiquement, tels que des agents tensioactifs anioniques, cationiques, non ioniques, amphotères, zwittérioniques ou leurs mélanges, des polymères anioniques, cationiques, non ioniques, amphotères, zwittérioniques, des agents épaississants minéraux, des agents antioxydants, des agents de pénétration, des agents séquestrants, des parfums, des tampons, des agents dispersants, des agents de conditionnement, des agents filmogènes, des céramides, des agents conservateurs, des agents stabilisants, des agents opacifiants.

Lorsque des tensioactifs sont présents, on utilise de préférence des tensioactifs non ioniques, anioniques ou amphotères, et de préférence des alkylsulfates, des alkyléthersulfates, des bétaïnes, des dérivés d'imidazolium, des alkylpyrrolidones, des éthers d'alcools gras oxyalkylénés ou glycérolés, des esters d'acides gras de monoalcools ou polyol éventuellement oxyalkylénés ou glycérolés. Plus particulièrement, leur teneur varie entre 0,01 et 30 % en poids par rapport au poids total de la composition, avantageusement, entre 0,1 et 20 % en poids et de préférence de 0,2 à 10% en poids du poids total de la composition.

La composition peut de plus comprendre un agent épaississant, et de préférence un système épaississant à base de polymères associatifs bien connus de l'homme de l'art et notamment de nature non ionique, anionique, cationique ou amphotère. A titre d'exemples, on peut citer tout particulièrement les homopolymères d'acide acrylique réticulés ; les homopolymères et copolymères réticulés d'acide 2-acrylamido-2-méthylpropane sulfonique partiellement ou totalement neutralisés ; les homopolymères ou copolymrèes d'acrylate d'ammonium ; les homopolymères ou copoylmèers de diméthylaminoéthyl-méthacrylate quaternisé ; les gommes de guar non ioniques, de biopolysaccharides d'origine microbienne (scléroglucane, xanthane), issues d'exudats végétaux (arabique, Ghatti, Karaya, Tragacanthe) ; les hydroxypropyl ou carboxyméthyl celluloses ; les pectines ; les alginates, ou leurs mélanges.
Si un tel agent est présent, sa teneur représente habituellement de 0,01 à 10% en poids, de préférence de 0,1 à 5% en poids, par rapport au poids total de la composition.

Il est de plus à noter que la composition peut comprendre un ou plusieurs agents de conditionnement tels que par exemple des cations, des silicones volatiles ou non, modifiées ou non, les huiles, les dérivés de polyuréthanes associatifs, les dérivés de celluloses associatifs, les dérivés de polyvinyllactames associatifs et les dérivés de polyacides insaturés associatifs, ou leurs mélanges.
Si un tel agent est présent, sa teneur représente habituellement de 0,0025 à 10% en poids, plus particulièrement 0,025 à 10% en poids, par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de liquides, de shampooings, de crèmes, de gels, ou sous toute autre forme appropriée.

Une forme particulièrement préférée selon la présente invention, la composition se trouve sous la forme d'un shampooing colorant et éclaircissant comprenant dans un milieu aqueux cosmétiquement acceptable.

Comme cela a été indiqué plus haut, le procédé de coloration avec effet éclaircissant selon l'invention, consiste à mettre en oeuvre les étapes suivantes :
a) on applique sur les fibres kératiniques humaines, une composition comprenant, dans un milieu cosmétiquement acceptable, au moins un colorant fluorescent soluble dans le milieu, pendant un temps suffisant pour développer la coloration et l'éclaircissement désirés,
b) on rince éventuellement les fibres,
c) éventuellement on lave au shampooing et on rince les fibres,
d) on sèche ou on laisse sécher les fibres.

Il est à noter que les termes fibres kératiniques humaines, signifient les cheveux. Plus particulièrement le procédé selon l'invention est très avantageux pour le traitement de cheveux pigmentés ou colorés artificiellement.
Au sens de l'invention, on entend par cheveux pigmentés ou colorés artificiellement, des cheveux dont la hauteur de ton est inférieure ou égale à 6 (blond foncé) et de préférence inférieure ou égale à 4 (châtain).

L'éclaircissement des cheveux est évalué par la 'hauteur de ton" qui caractérise le degré
ou le niveau d'éclaircissement. La notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage "Sciences des traitements capillaires" de Charles ZVIAK 1988, Ed.Masson, pp.215 et 278.
Les hauteurs de ton s'échelonnent de 1 (noir) à 10 (blond clair), une unité correspondant à un ton ; plus le chiffre est élevé et plus la nuance est claire.

Préalablement au procédé de coloration avec effet éclaircissant, les fibres ont subi un procédé de déformation permanente.

Selon une première possibilité, ledit traitement de déformation consiste à mettre en oeuvre les étapes suivantes :
(*) on applique sur les fibres kératiniques, en les lissant, une composition aqueuse alcaline dont le pH est d'au moins 10, et on laisse pauser pendant la durée suffisante pour la mise en forme,
(**) on rince éventuellement les fibres, on les lave au shampoing et on les rince à nouveau, on sèche éventuellement.

La composition aqueuse alcaline est une solution aqueuse comprenant au moins un hydroxyde de métal alcalin, de préférence de sodium.
Il est à noter que ladite composition se trouve de manière avantageuse sous la forme d'une émulsion huile dans eau, la phase huileuse étant constituée par exemple d'huile de vaseline.
La concentration en agent alcalin est en général comprise entre 1 et 4 % en poids de la composition mise en oeuvre lors de cette étape (*).
Habituellement l'étape (*) est réalisée à température ambiante.

Le temps de pause est en général compris entre 3 et 30 minutes, de préférence entre 5 et 15 minutes.

Selon une deuxième possibilité, le traitement de déformation consiste à mettre en oeuvre les étapes suivantes :
(*) on applique sur les fibres kératiniques, une composition réductrice comprenant dans un milieu cosmétiquement acceptable, au moins un agent réducteur, et on laisse pauser pendant la durée suffisante à la mise en forme,
(**) on rince les fibres et on applique une composition oxydante, pendant une durée suffisante à la fixation de la forme,
(***) on rince éventuellement les fibres, on les lave au shampoing et on les rince à nouveau, on sèche éventuellement.

La composition mise en oeuvre dans l'étape (*) de ce procédé, comprend de manière habituelle, à titre d'agent réducteur des thiols tels que l'acide thioglycolique et l'acide thiolactique, leurs sels et leurs esters, la cystéine, la cystéamine et leurs dérivés, les sulfites et des bisulfites, notamment de métal alcalin, alcalino-terreux ou d'ammonium, et leur mélanges.

La teneur en agent réducteur est généralement comprise entre 1 et 30% en poids, et de préférence entre 5 et 20% en poids par rapport au poids de la composition réductrice.

Généralement, le milieu de cette composition comprend de l'eau ou un mélange eau et solvant cosmétiquement acceptable. Les solvants listés dans le cadre de la composition comprenant le colorant fluorescent peuvent être utilisés.
La teneur en solvant est plus particulièrement d'au plus 20 % en poids par rapport au poids de la composition réductrice.

La composition réductrice peut aussi comprendre des additifs usuels tels que des agents tensioactifs non ioniques, anioniques, cationiques ou amphotères et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés, ainsi que d'autres tensio-actifs non-ioniques du type hydroxypropyléther.
Lorsque la composition réductrice ce type d'additif, sa teneur est général de moins de 30% en poids, et de préférence comprise entre 0,5 et 10% en poids, par rapport au poids de la composition réductrice.

La composition réductrice peut se présenter sous la forme d'une lotion, épaissie ou non, d'une crème, d'un gel, ou de toute autre forme appropriée.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes sous forme d'émulsions "lourdes", obtenues par exemple en émulsionant une phase aqueuse, comprenant notamment l'agent réducteur, et une phase huileuse (huile végétale, huile de paraffine, esters d'acides gras, cire, par exemple).
On peut également utiliser des liquides ou des gels contenant des agents épaississants tels que des polymères ou des copolymères carboxyvinyliques qui "collent" les cheveux et les maintiennent dans la position lisse pendant le temps de pose.

Si l'opération est destinée à friser les fibres, ces dernières sont mises sous tension au moyen de bigoudis, avant, pendant ou après l'application de la composition.

Le temps de pause est en général compris entre 3 et 30 minutes, de préférence entre 5 et 15 minutes.

La composition oxydante mise en oeuvre lors de l'étape (**) comprend de manière classique au moins un oxydant, en général de l'eau oxygénée, un bromate alcalin, un persel ou un polythionate.

Le pH de la composition oxydante est généralement compris entre 2 et 10.

Le temps de pause est en général compris entre 3 et 30 minutes, de préférence entre 5 et 15 minutes.

Le plus souvent, les cheveux imprégnés de la composition oxydante sont rincés soigneusement, généralement à l'eau. On sépare, avant ou après le rinçage, la fibre kératinique des moyens nécessaires à sa mise sous tension.

Le procédé de coloration peut être effectué sur fibres sèches ou humides, immédiatement ou non après le procédé de déformation.

Selon une première variante, le procédé de coloration conforme à l'invention, consiste à appliquer sur les fibres, et notamment les cheveux, au moins une composition telle que définie précédemment, pendant un temps suffisant pour développer la coloration et l'éclaircissement désirés, après quoi on rince, on lave éventuellement au shampooing, on rince à nouveau et on sèche.

Le temps nécessaire au développement de la coloration et à l'obtention de l'effet éclaircissant sur les fibres, notamment les cheveux, est d'environ 5 à 60 minutes et plus particulièrement d'environ 5 à 40 minutes.

La température nécessaire au développement de la coloration et à l'obtention de l'effet éclaircissant est généralement comprise entre la température ambiante (15 à 25°C) et 80°C et plus particulièrement entre 15 et 40 °C.

Selon une deuxième variante de ces procédés de teinture conformes à l'invention, on applique sur les fibres, et notamment les cheveux au moins une composition telle que définie précédemment sans rinçage final.

Il est à noter que la composition selon l'invention, si elle est utilisée pour traiter des fibres kératiniques, telles que des cheveux châtains par exemple, permet d'atteindre les résultats suivants :
Si l'on mesure la réflectance des cheveux lorsqu'on les irradie avec de la lumière visible dans la gamme de longueurs d'onde allant de 400 à 700 nanomètres, et que l'on compare les courbes de réflectance en fonction de la longueur d'onde, des cheveux traités avec la composition de l'invention et des cheveux non traités, on constate que la courbe de réflectance correspondant aux cheveux traités, dans une gamme de longueur d'onde allant de 500 à 700 nanomètres, est supérieure à celle correspondant aux cheveux non traités.
Cela signifie que, dans la gamme de longueur d'onde allant de 500 à 700 nanomètres, et de préférence de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%.
Il est précisé toutefois qu'il peut exister dans la gamme de longueur d'onde allant de 500 à 700 nanomètres, et de préférence de 540 à 700 nanomètres, une ou plusieurs plages
où la courbe de réflectance correspondant aux fibres traitées est soit superposable, soit inférieure à la courbe de réflectance correspondant aux fibres non traitées.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

En outre, et de préférence, la composition selon l'invention est susceptible d'éclaircir les cheveux et la peau dans une nuance qui, chiffrée dans le système C.I.E.L L*a*b* présente une variable b* supérieure ou égale à 6, avec un rapport b*/valeur absolue de a*, supérieur à 1,2 selon le test de sélection décrit ci-dessous.

### Test de sélection

La composition est appliquée sur des fibres kératiniques châtain, plus particulièrement des cheveux, à raison de 10 grammes de composition pour 1 gramme de fibres châtain. La composition est étalée de façon à recouvrir l'ensemble des fibres. On laisse la composition agir pendant 20 minutes à température ambiante (20 à 25°C). Les fibres sont ensuite rincées à l'eau puis lavées avec un shampooing à base de lauryléther sulfate. Elles sont ensuite séchées. On mesure alors les caractéristiques spectrocolorimétriques des fibres pour en déterminer les coordonnées L*a*b*.
Dans le système C.I.E.L L*a*b*, a* et b* indiquent deux axes de couleurs, a* indique l'axe de couleur vert/rouge (+a* est rouge, -a* est vert) et b* l'axe de couleur bleu/jaune (+b* est jaune et -b* est bleu) ; des valeurs proches de zéro pour a* et b* correspondent à des nuances grises.

L'exemple qui suit est destiné à illustrer l'invention sans pour autant en limiter la portée.

### EXEMPLE

On applique sur une mèche de cheveux de type africain, en la lissant, une émulsion eau dans huile de vaseline comprenant une solution d'hydroxyde de sodium à 3 % en poids, pendant 15 minutes, après quoi on rince soigneusement la mèche.

On applique ensuite une composition suivante selon l'invention :

| | |
|---|---|
| Composé fluorescent (*) | 0,6 % |
| N-cocoyl amidoéthyl N-éthoxycarboxyméthyl glycinate de sodium | 2 % |
| Hexylèneglycol | 7 % |
| Eau distillée qsp | 100 g |

| | |
|---|---|
| Les pourcentages sont exprimés en poids de matière active. (*) Composé fluorescent : 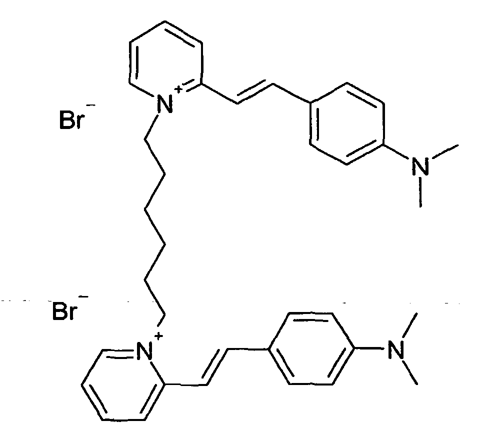 | |

Ce composé est obtenu selon la méthode ci-dessous :
On fait réagir 93 g de 2-picoline avec 120g de 1,6 dibromohexane dans le diméthylformamide à 110°C pendant 5 heures.
On récupère le produit précipité, et on le filtre.
On solubilise 109 g du produit obtenu précédemment dans du méthanol et l'on ajoute 82,82 g de p-diméthylaminobenzaldéhyde en deux fois, en présence de pyrrolidine.
On laisse ensuite pendant 30 minutes.
On récupère le produit sous forme précipitée.

Analyse par spectroscopie de masse : 266.
Analyse élémentaire : C : 62,43 % ; H : 6,40 % ; Br : 23,07 % ; N : 8,09 %.
La formule est la suivante C₃₆H₄₄N₄.2Br.

La composition est appliquée sur la mèche traitée avec un temps de pose de 20 minutes. Les mèches sont ensuite rincées et un séchées au casque pendant 30 minutes.

On obtient un net effet d'éclaircissement de la mèche de cheveux.

## Revendications

1. Procédé pour colorer avec un effet éclaircissant les fibres kératiniques humaines ayant subi antérieurement un procédé de déformation permanente, **caractérisé en ce que** l'on met en oeuvre les étapes suivantes :
a) on applique sur lesdites fibres, une composition comprenant, dans un milieu cosmétiquement acceptable, au moins un colorant fluorescent soluble dans le milieu, pendant un temps suffisant pour développer la coloration et l'éclaircissement désirés, le colorant fluorescent étant une molécule qui colore par elle-même, et donc absorbe la lumière du spectre visible, mais qui transforme l'énergie absorbée en lumière fluorescente de plus grande longueur d'onde émise dans la partie visible de spectre,
b) on rince éventuellement les fibres,
c) éventuellement on lave au shampooing et on rince les fibres,
d) on sèche ou on laisse sécher les fibres.

2. Procédé selon la revendication précédente, **caractérisé en ce que** la composition comprend au moins un colorant fluorescent présentant un maximum de réflectance se situant dans la gamme de longueur d'onde allant de 500 à 650 nanomètres, et de préférence dans la gamme de longueur d'onde allant de 550 à 620 nanomètres.

3. Procédé selon l'une des revendications précédentes, **caractérisé en ce que** la composition comprend au moins un colorant fluorescent choisi parmi ceux des familles suivantes : les naphtalimides ; les coumarines cationiques ou non ; les xanthénodiquinolizines ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges.

4. Procédé selon l'une des revendications précédentes, **caractérisé en ce que** la composition comprend au moins un colorant fluorescent choisis parmi les composés suivants : 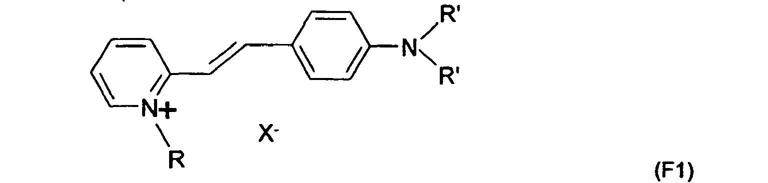 formule dans laquelle R représente un radical méthyle ou éthyle; R' représente un radical méthyle, X⁻ un anion du type iodure, sulfate, méthosulfate ; 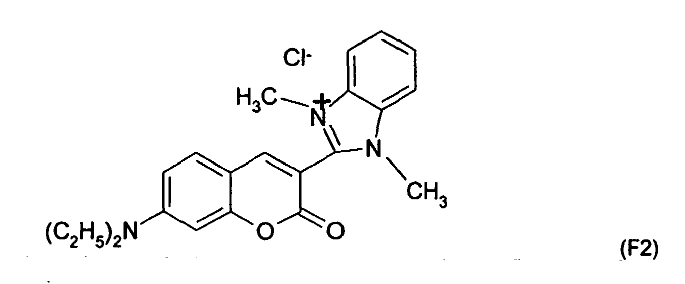 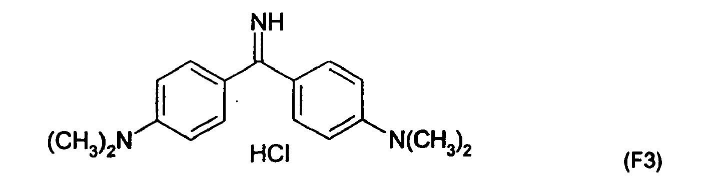 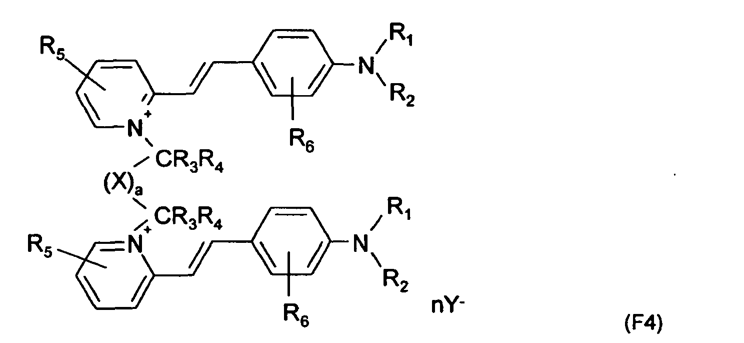 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

5. Procédé selon l'une quelconque des revendications précédentes, **caractérisé en ce que** la composition une concentration pondérale en colorant(s) fluorescent(s) comprise entre 0,01 et 20% en poids, plus particulièrement comprise entre 0,05 à 10% en poids, de préférence comprise entre 0,1 à 5% en poids, par rapport au poids total de la composition.

6. Procédé selon l'une des revendications précédentes, **caractérisé en ce que** la composition comprend au moins un tensioactif non ionique, anionique ou amphotère.

7. Procédé selon la revendication précédente, **caractérisé en ce que** la composition comprend une teneur en tensioactif variant entre 0,01 à 30 % en poids par rapport au poids total de la composition.

8. Procédé selon l'une quelconque des revendications précédentes, **caractérisé en ce que** la composition comprend au moins un colorant direct additionnel non fluroescent de nature non ionique, cationique ou anionique.

9. Procédé selon la revendications précédente, **caractérisé en ce que** les colorants directs additionnels sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ainsi que les colorants dérivés du triarylméthane, ou leurs mélanges.

10. Procédé selon l'une des revendications 9 ou 10, **caractérisé en ce que** la composition comprend une teneur en colorants directs additionnels comprise entre 0,0005 et 12 % en poids, de préférence entre 0,005 et 6 % en poids, par rapport au poids total de la composition.

11. Procédé selon l'une quelconque des revendications précédentes, **caractérisé en ce que** la composition se présente sous la forme d'un shampooing éclaircissant et colorant.

12. Procédé selon l'une quelconque des revendications précédentes, **caractérisé en ce que** la composition est appliquée sur des fibres kératiniques ayant fait l'objet d'une déformation permanente préalable, consistant à mettre en oeuvre les étapes suivantes :
(*) on applique sur les fibres kératiniques, en les lissant, une composition aqueuse alcaline dont le pH est d'au moins 10, et on laisse pauser pendant la durée suffisante à la mise en forme,
(**) on rince éventuellement les fibres, on les lave au shampoing et on les rince à nouveau, on sèche éventuellement.

13. Procédé selon l'une quelconque des revendications 1 à 11, **caractérisé en ce que** la composition est appliquée sur des fibres kératiniques ayant fait l'objet d'une déformation permanente préalable, consistant à mettre en oeuvre les étapes suivantes :
(*) on applique sur les fibres kératiniques, une composition réductrice comprenant dans un milieu cosmétiquement acceptable, au moins un agent réducteur, et on laisse pauser pendant la durée suffisante à la mise en forme,
(**) on rince les fibres et on applique une composition oxydante, pendant une durée suffisante à la fixation de la forme,
(***) on rince éventuellement les fibres, on les lave au shampoing et on les rince à nouveau, on sèche éventuellement.

14. Procédé selon l'une quelconque des revendications précédentes, **caractérisé en ce que** la composition est appliquée sur des cheveux présentant une hauteur de ton inférieure ou égale à 6 et de préférence inférieure ou égale à 4.

15. Procédé selon l'une des revendications précédentes, **caractérisé en ce que** les fibres kératiniques humaines sont pigmentées ou colorées artificiellement.

## Claims

1. Process for dyeing, with a lightening effect, human keratin fibres that have previously been subjected to a permanent reshaping process, **characterized in that** the following steps are performed:
a) a composition comprising, in a cosmetically acceptable medium, at least one fluorescent dye that is soluble in the medium is applied to the said fibres, for a time that is sufficient to develop the desired coloration and lightening, the fluorescent dye being a molecule that colours by itself, and thus absorbs light in the visible spectrum, but which converts the absorbed energy into fluorescent light of a longer wavelength emitted in the visible region of the spectrum,
b) the fibres are optionally rinsed,
c) the fibres are optionally washed with shampoo and rinsed,
d) the fibres are dried or are left to dry.

2. Process according to the preceding claim, **characterized in that** the composition comprises at least one fluorescent dye which has a reflectance maximum that is in the wavelength range from 500 to 650 nanometres and preferably in the wavelength range from 550 to 620 nanometres.

3. Process according to either of the preceding claims, **characterized in that** the composition comprises at least one fluorescent dye chosen from those of the following families: naphthalimides; cationic or non-cationic coumarins; xanthenodiquinolizines; azaxanthenes; naphtholactams; azlactones; oxazines; thiazines; dioxazines; monocationic or polycationic fluorescent dyes of azo, azomethine or methine type, alone or as mixtures.

4. Process according to one of the preceding claims, **characterized in that** the composition comprises at least one fluorescent dye chosen from the following compounds: 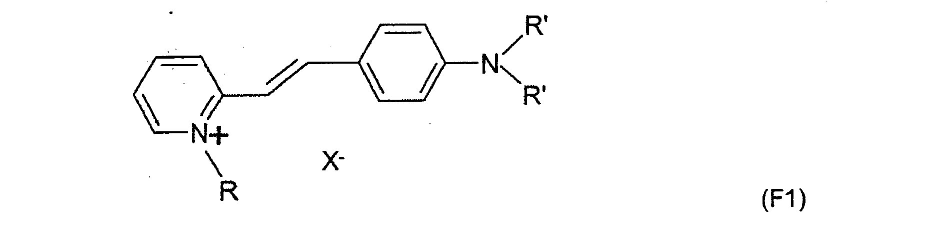 in which formula R represents a methyl or ethyl radical; R' represents a methyl radical and X⁻ represents an anion such as iodide, sulphate or methosulphate; 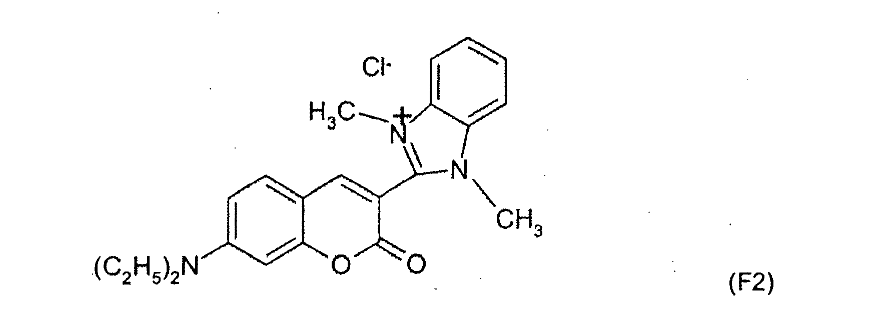 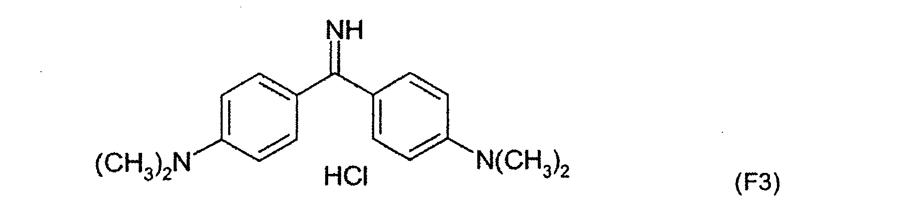 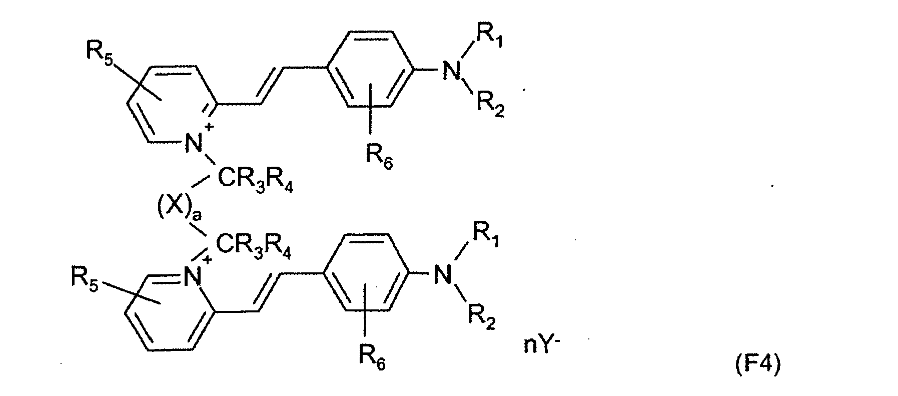 in which:
R₁ and R₂, which may be identical or different, represent:
• a hydrogen atom;
• a linear or branched alkyl radical containing 1 to 10 carbon atoms and preferably from 1 to 4 carbon atoms, optionally interrupted and/or substituted with at least one hetero atom and/or group comprising at least one hetero atom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one hetero atom and/or group comprising at least one hetero atom and/or substituted with at least one halogen atom;
• R₁ and R₂ may optionally be linked so as to form a heterocycle with the nitrogen atom and may comprise one or more other hetero atoms, the heterocycle optionally being substituted with at least one linear or branched alkyl radical preferably containing from 1 to 4 carbon atoms and optionally being interrupted and/or substituted with at least one hetero atom and/or group comprising at least one hetero atom and/or substituted with at least one halogen atom;
• R₁ or R₂ may optionally be engaged in a heterocycle comprising the nitrogen atom and one of the carbon atoms of the phenyl group bearing the said nitrogen atom;
R₃ and R₄, which may be identical or different, represent a hydrogen atom or an alkyl radical containing 1 to 4 carbon atoms;
R₅, which may be identical or different, represent a hydrogen atom, a halogen atom or a linear or branched alkyl radical containing 1 to 4 carbon atoms, optionally interrupted with at least one hetero atom;
R₆, which may be identical or different, represent a hydrogen atom; a halogen atom; a linear or branched alkyl radical containing 1 to 4 carbon atoms, optionally substituted and/or interrupted with at least one hetero atom and/or group bearing at least one hetero atom and/or substituted with at least one halogen atom;
X represents:
• a linear or branched alkyl radical containing 1 to 14 carbon atoms or an alkenyl radical containing 2 to 14 carbon atoms, optionally interrupted and/or substituted with at least one hetero atom and/or group containing at least one hetero atom and/or substituted with at least one halogen atom;
• a 5- or 6-membered heterocyclic radical optionally substituted with at least one linear or branched alkyl radical containing 1 to 14 carbon atoms, optionally substituted with at least one hetero atom; with at least one linear or branched aminoalkyl radical containing 1 to 4 carbon atoms, optionally substituted with at least one hetero atom; with at least one halogen atom;
• a fused or non-fused aromatic or diaromatic radical, optionally separated with an alkyl radical containing 1 to 4 carbon atoms, the aryl radical(s) optionally being substituted with at least one halogen atom or with at least one alkyl radical containing 1 to 10 carbon atoms optionally substituted and/or interrupted with at least one hetero atom and/or group bearing at least one hetero atom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound.

5. Process according to any one of the preceding claims, **characterized in that** the composition has a weight concentration of fluorescent dye(s) of between 0.01% and 20% by weight, more particularly between 0.05% and 10% by weight and preferably between 0.1% and 5% by weight relative to the total weight of the composition.

6. Process according to one of the preceding claims, **characterized in that** the composition comprises at least one nonionic, anionic or amphoteric surfactant.

7. Process according to the preceding claim, **characterized in that** the composition has a surfactant content ranging from 0.01% to 30% by weight relative to the total weight of the composition.

8. Process according to any one of the preceding claims, **characterized in that** the composition comprises at least one additional non-fluorescent direct dye of nonionic, cationic or anionic nature.

9. Process according to the preceding claim, **characterized in that** the additional direct dyes are chosen from nitrobenzene dyes, azo dyes, anthraquinone dyes, naphthoquinone dyes, benzoquinone dyes, phenothiazine dyes, indigoid dyes, xanthene dyes, phenanthridine dyes, phthalocyanin dyes and triarylmethane-based dyes, or mixtures thereof.

10. Process according to either of Claims 9 and 10, **characterized in that** the composition comprises an amount of additional direct dyes of between 0.0005% and 12% by weight and preferably between 0.005% and 6% by weight relative to the total weight of the composition.

11. Process according to any one of the preceding claims, **characterized in that** the composition is in the form of a lightening dyeing shampoo.

12. Process according to any one of the preceding claims, **characterized in that** the composition is applied to keratin fibres that have been permanently reshaped beforehand, which consists in performing the following steps:
(*) an alkaline aqueous composition with a pH of at least 10 is applied to the keratin fibres, while smoothing them out, and is left on the fibres for a time that is sufficient to shape them,
(**) the fibres are optionally rinsed, washed with shampoo, rinsed again and optionally dried.

13. Process according to any one of Claims 1 to 11, **characterized in that** the composition is applied to keratin fibres that have been permanently reshaped beforehand, which consists in performing the following steps:
(*) a reducing composition comprising, in a cosmetically acceptable medium, at least one reducing agent is applied to the keratin fibres and is left on the fibres for a time that is sufficient to shape them,
(**) the fibres are rinsed and an oxidizing composition is applied, for a time that is sufficient to fix the shape,
(***) the fibres are optionally rinsed, washed with shampoo, rinsed again and optionally dried.

14. Process according to any one of the preceding claims, **characterized in that** the composition is applied to hair with a tone height of less than or equal to 6 and preferably less than or equal to 4.

15. Process according to one of the preceding claims, **characterized in that** the human keratin fibres are artificially dyed or pigmented.

## Patentansprüche

1. Verfahren zum Färben von menschlichen Keratinfasern, die zuvor dauerhaft verformt wurden, mit aufhellender Wirkung, **dadurch gekennzeichnet, dass** die folgenden Schritte durchgeführt werden:
a) auf die Fasern wird eine Zusammensetzung, die in einem kosmetisch akzeptablen Medium mindestens einen in dem Medium löslichen fluoreszierenden Farbstoff enthält, während einer Zeitspanne aufgebracht, die ausreichend ist, um die gewünschte Färbung und die gewünschte Aufhellung zu erzielen, wobei der fluoreszierende Farbstoff ein Molekül ist, das selbst farbig ist und daher Licht im sichtbaren Bereich absorbiert, jedoch die absorbierte Energie in Fluoreszenzlicht mit größerer Wellenlänge im sichtbaren Bereich des Spektrums umwandelt.
b) die Fasern werden gegebenenfalls gespült,
c) die Fasern werden gegebenenfalls mit Haarwaschmittel gewaschen und gespült,
d) die Fasern werden getrocknet oder trocknen gelassen.

2. Verfahren nach dem vorhergehenden Anspruch, **dadurch gekennzeichnet, dass** die Zusammensetzung mindestens einen fluoreszierenden Farbstoff enthält, der einen maximalen Reflexionsgrad im Wellenlängenbereich von 500 bis 650 nm und vorzugsweise im Wellenlängenbereich von 550 bis 620 nm aufweist.

3. Verfahren nach einem der vorhergehenden Ansprüche, **dadurch gekennzeichnet, dass** die Zusammensetzung mindestens einen fluoreszierenden Farbstoff enthält, der aus den folgenden Gruppen ausgewählt ist: Naphthalimiden; kationischen oder nicht kationischen Cumarinen; Xanthenodichinolizinen; Azaxanthenen; Naphtholactamen; Azlactonen; Oxazinen; Thiazinen; Dioxazinen; mono- oder polykationischen fluoreszierenden Farbstoffen vom Azotyp, Azomethintyp oder Methintyp, wobei diese Farbstoffe einzeln oder im Gemisch vorliegen.

4. Verfahren nach einem der vorhergehenden Ansprüche, **dadurch gekennzeichnet, dass** die Zusammensetzung mindestens einen fluoreszierenden Farbstoff enthält, der unter den folgenden Verbindungen ausgewählt ist: 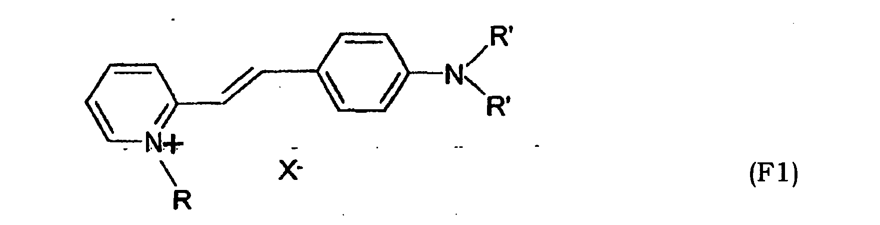 wobei in der Formel R Methyl oder Ethyl bedeutet; R' eine Methylgruppe ist, X⁻ ein Anion vom Typ Iodid, Sulfat, Methosulfat ist; 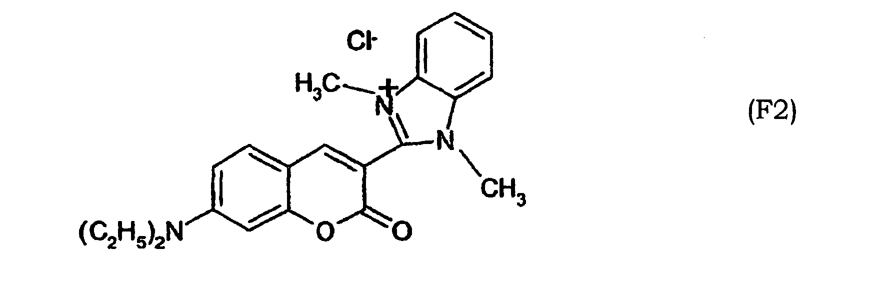 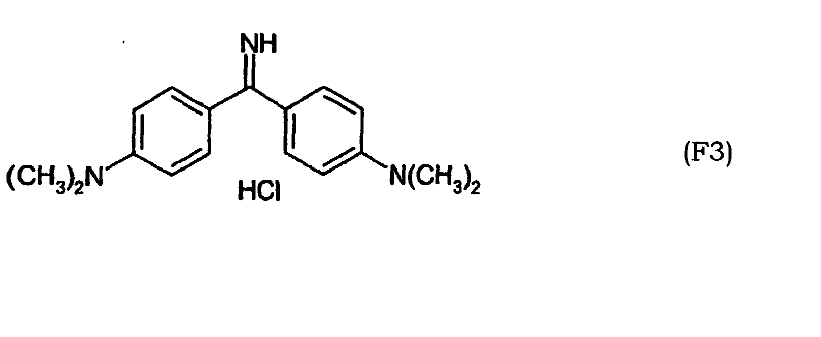 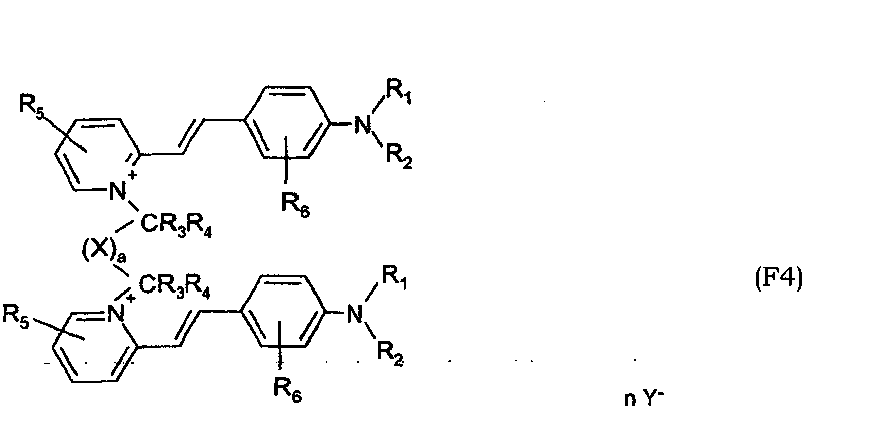 worin bedeuten:
die Gruppen R₁ und R₂, die gleich oder verschieden sind:
· ein Wasserstoffatom;
· eine geradkettige oder verzweigte Alkylgruppe mit 1 bis 10 Kohlenstoffatomen und vorzugsweise 1 bis 4 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· eine Aryl- oder Arylalkylgruppe, wobei die Arylgruppe 6 Kohlenstoffatome und die Alkylgruppe 1 bis 4 Kohlenstoffatome besitzt, wobei die Arylgruppe gegebenenfalls mit einer oder mehreren geradkettigen oder verzweigten Alkylgruppen mit 1 bis 4 Kohlenstoffatomen substituiert ist, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert sind und/oder mit mindestens einem Halogenatom substituiert sind;
· die Gruppen R₁ und R₂ können gegebenenfalls so verbunden sein, dass sie mit dem Stickstoffatom einen Heterocyclus bilden, wobei ein oder mehrere weitere Heteroatome enthalten sein können, wobei der Heterocyclus gegebenenfalls mit mindestens einer geradkettigen oder verzweigten Alkylgruppe substituiert ist, die vorzugsweise 1 bis 4 Kohlenstoffatome enthält und gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· die Gruppe R₁ oder R₂ kann gegebenenfalls in einen Heterocyclus eingebunden sein, der das Stickstoffatom und ein Kohlenstoffatom des Phenylrings, der das Stickstoffatom trägt, enthält;
die Gruppen R₃ und R₄, die identisch oder verschieden sind, ein Wasserstoffatom oder eine Alkylgruppe mit 1 bis 4 Kohlenstoffatomen;
die Gruppen R₅, die gleich oder verschieden sind, ein Wasserstoffatom; ein Halogenatom; eine geradkettige oder verzweigte Alkylgruppe mit 1 bis 4 Kohlenstoffatomen, die gegebenenfalls durch mindestens ein Heteroatom unterbrochen ist;
die Gruppen R₆, die gleich oder verschieden sind, ein Wasserstoffatom; ein Halogenatom; eine geradkettige oder verzweigte Alkylgruppe mit 1 bis 4 Kohlenstoffatomen, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist und/oder mit mindestens einem Halogenatom substituiert ist;
X bedeutet:
· eine geradkettige oder verzweigte Alkylgruppe mit 1 bis 14 Kohlenstoffatomen oder eine Alkenylgruppe mit 2 bis 14 Kohlenstoffatomen, die gegebenenfalls durch mindestens ein Heteroatom und/oder eine Gruppe, die mindestens ein Heteroatom enthält, unterbrochen und/ oder mit mindestens einem Heteroatom und/oder einer Gruppe, die mindestens ein Heteroatom enthält, substituiert und/ oder mit mindestens einem Halogenatom substituiert ist;
· eine 5- oder 6-gliedrige heterocyclische Gruppe, die gegebenenfalls mit mindestens einer geradkettigen oder verzweigten Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom substituiert ist;
· eine aromatische Gruppe oder eine kondensierte oder nicht kondensierte, diaromatische Gruppe, die gegebenenfalls durch eine Alkylgruppe mit 1 bis 4 Kohlenstoffatomen separiert wird, wobei die Arylgruppe(n) gegebenenfalls mit mindestens einem Halogenatom oder mit mindestens einer Alkylgruppe mit 1 bis 10 Kohlenstoffatomen substituiert sind, die gegebenenfalls mit mindestens einem Heteroatom und/oder eine Gruppe, die mindestens ein Heteroatom enthält, substituiert und/oder durch mindestens ein Heteroatom und/oder eine Gruppe, die mindestens ein Heteroatom enthält, unterbrochen ist;
· eine Dicarbonylgruppe;
· wobei die Gruppe X eine oder mehrere kationische Ladungen
umfassen kann;
wobei a 0 oder 1 beträgt;
die Gruppen Y⁻ , die gleich oder verschieden sind, ein organisches oder anorganisches Anion;
wobei n eine ganze Zahl von mindestens 2 und höchstens der Anzahl der in der fluoreszierenden Verbindung vorliegenden kationischen Ladungen ist.

5. Verfahren nach einem der vorhergehenden Ansprüche, **dadurch gekennzeichnet, dass** der oder die fluoreszierende(n) Farbstoff(e) in der Zusammensetzung in einer Konzentration von 0,01 bis 20 Gew.-%, insbesondere 0,05 bis 10 Gew.-% und vorzugsweise 0,1 bis 5 Gew.-%, bezogen auf das Gesamtgewicht der Zusammensetzung, enthalten sind.

6. Verfahren nach einem der vorhergehenden Ansprüche, **dadurch gekennzeichnet, dass** die Zusammensetzung mindestens einen nichtionischen, anionischen oder amphoteren grenzflächenaktiven Stoff enthält.

7. Verfahren nach dem vorhergehenden Anspruch, **dadurch gekennzeichnet, dass** der Mengenanteil des grenzflächenaktiven Stoffes 0,01 bis 30 Gew.-%, bezogen auf das Gesamtgewicht der Zusammensetzung, ausmacht.

8. Verfahren nach einem der vorhergehenden Ansprüche, **dadurch gekennzeichnet, dass** sie ferner mindestens einen zusätzlichen, nicht fluoreszierenden Direktfarbstoff vom nichtionischen, kationischen oder anionischen Typ enthält.

9. Verfahren nach dem vorhergehenden Anspruch, **dadurch gekennzeichnet, dass** die zusätzlichen Direktfarbstoffe unter den nitrierten Benzolfarbstoffen, Azofarbstoffen, AnthrachinonFarbstoffen, Naphthochinon-Farbstoffen, Benzochinon-Farbstoffen, Phenotiazin-Farbstoffen, Indigoiden, Xanthen-Farbstoffen, Phenanthridin-Farbstoffen, Phthalocyaninen sowie den von Triarylmethan abgeleiteten Farbstoffen oder deren Gemischen ausgewählt sind.

10. Verfahren nach einem der Ansprüche 9 oder 10, **dadurch gekennzeichnet, dass** der oder die zusätzliche(n) Direktfarbstoff(e) 0,0005 bis 12 Gew.-% und vorzugsweise 0,005 bis 6 Gew.-% des Gesamtgewichts der Zusammensetzung ausmachen.

11. Verfahren nach einem der vorhergehenden Ansprüche, **dadurch gekennzeichnet, dass** die Zusammensetzung in Form eines auf hellenden und färbenden Haarwaschmittels vorliegt.

12. Verfahren nach einem der vorhergehenden Ansprüche, **dadurch gekennzeichnet, dass** die Zusammensetzung auf Keratinfasern aufgetragen wird, die zuvor einer dauerhaften Verformung unterzogen wurden, die aus den folgenden Schritten besteht:
(*) auf die Keratinfasern wird eine alkalische wässrige Lösung aufgetragen, deren pH-Wert mindestens 10 beträgt und während einer Zeitspanne einwirken gelassen, die für die Formgebung ausreichend ist, wobei die Fasern gleichzeitig geglättet werden,
(**) die Fasern werden gegebenenfalls gespült, mit Haarwaschmittel gewaschen, nochmals gespült und gegebenenfalls getrocknet.

13. Verfahren nach einem der Ansprüche 1 bis 11, **dadurch gekennzeichnet, dass** die Zusammensetzung auf Keratinfasern aufgetragen wird, die zuvor einer dauerhaften Verformung unterzogen wurden, die aus den folgenden Schritten besteht:
(*) auf die Keratinfasern wird eine reduzierende Lösung aufgetragen, die in einem kosmetisch akzeptablen Medium mindestens ein Reduktionsmittel enthält, und während einer Zeitspanne einwirken gelassen, die für die Formgebung ausreichend ist, (**) die Fasern werden gespült und man bringt während einer Zeitspanne, die für die Fixierung der Form ausreichend ist, eine oxidierende Zusammensetzung auf,
(***) die Fasern werden gegebenenfalls gespült, mit Haarwaschmittel gewaschen, nochmals gespült und gegebenenfalls getrocknet.

14. Verfahren nach einem der vorhergehenden Ansprüche, **dadurch gekennzeichnet, dass** die Zusammensetzung auf Haare aufgetragen wird, die einen Farbton von 6 oder darunter und vorzugsweise 4 oder darunter aufweisen.

15. Verfahren nach einem der vorhergehenden Ansprüche, **dadurch gekennzeichnet, dass** die menschlichen Keratinfasern pigmentiert oder künstlich gefärbt sind.
